# EUROPÄISCHE PATENTSCHRIFT

(11) **EP 0 743 062 B1**
(45) Veröffentlichungstag und Bekanntmachung des Hinweises auf die Patenterteilung: **16.08.2001**
(21) Anmeldenummer: 96810285.5
(22) Anmeldetag: 06.05.1996
(51) Int. Cl.: A61K 9/00, A61K 35/78

(54) **Verfahren zum Herstellen eines Brausepräparats und Brausepräparat**
Effervescent preparation and production method thereof
Préparation effervescente et son procédé de production

(30) Priorität: 17.05.1995 CH 144195; 14.03.1996 CH 67296
(43) Veröffentlichungstag der Anmeldung: 20.11.1996
(73) Patentinhaber: Greither, Peter, CH-9533 Kirchberg (CH)
(72) Erfinder: Engel, Dieter Wolfgang, 9523 Züberwangen (CH); Greither, Peter, 9533 Kirchberg (CH)
(74) Vertreter: Wenger, René

(56) Entgegenhaltungen:
- EP-A- 0 531 155

## Beschreibung

Die Erfindung betrifft ein Verfahren zum Herstellen eines Brausepräparats, als Träger eines Arzneimittels oder Genussmittels auf pflanzlicher Basis. Brausetabletten oder auch Granulate als lagerfähige Träger für Vitaminpräparate, Analgetika oder andere Arzneimittel sind seit langem bekannt.

Die bisher angebotenen pflanzlichen Arzneimittel werden jedoch vorwiegend in fester Darreichungsform als Schluck-oder Kautabletten, als Pastillen, Kapseln oder in flüssiger Form als Tropfen, Tinkturen oder Sirupe angeboten. Die pflanzlichen Wirkstoffe werden dabei als getrocknete Pulver oder in Form von polaren oder unpolaren Trocken- oder Spissumextrakten eingesetzt. Die mit diesem Verfahren hergestellten getrockneten Pflanzenauszüge waren bisher jedoch nicht auf befriedigende Weise durch Resuspension in eine flüssige Lösung überzuführen, da die Extrakte zu einer Verschleimung führen. Die bisher im Markt befindlichen Frischpflanzensäfte, zu denen auch die Gemüsesäfte zählen, verfügen in der Regel über einen Trockenanteil zwischen 2% und 20%. Durch den schlechten Geschmack einiger Frischpflanzensäfte ist die Akzeptanz beim Verbraucher nicht gegeben. Anderseits bleiben bestimmte Wirkstoffe nur im Frischpflanzensaft erhalten, während sie bei den Extraktionsverfahren zur Gewinnung von Trockenextrakten verlorengehen oder verändert werden. In der Regel finden beim Trocknen der Droge irreversible Fermentationen, Hydrolysevorgänge, Kondensationen, Oxidationen oder Verluste an flüchtigen Bestandteilen, sowie andere Veränderungen statt.

Durch die WO 90/03179 ist ein Brausepräparat als Träger für ein analgetisches, Salizylsäure bildendes Mittel bekannt geworden, wobei das Mittel ein wasserlöslicher Weidenrindenextrakt mit hohem Wirkstoffgehalt ist. Damit sollen die negativen Nebenwirkungen der synthetisch hergestellten Salizylsäure beseitigt werden. Der Weidenrindenextrakt wird dabei jedoch offensichtlich aus getrockneten Pflanzen durch Extraktion gewonnen, wobei Salicin in ausreichender Menge und Reinheit anfällt. Ein sinnvoller Ersatz für Frischpflanzensäfte wird dadurch jedoch nicht nahegelegt.

Durch die EP-A 531 155 ist die Verwendung der schwarzen Johannisbeere als Heilmittel in der Form von Saft, Konzentrat oder Trockenextrakt bekannt geworden. Als alternative Darreichungsform werden auch Brausetabletten erwähnt. Die Publikation enthält jedoch ebenfalls keine Anregung zur schonenden Aufschliessung der Pflanzenzellen und es muss davon ausgegangen werden, dass zur Herstellung der Brausetabletten konventionelle Trockenextrakte verwendet werden.

Es ist daher eine Aufgabe der Erfindung, ein Verfahren der eingangs genannten Art zu schaffen, mit dessen Hilfe Frischpflanzensäfte ohne Beeinträchtigung der darin enthaltenen Wirkstoffe oder Geschmacksstoffe in ein lagerfähiges Präparat umgewandelt werden können, das jederzeit wieder in eine Lösung übergeführt werden kann, ohne dass dabei eine Verschleimung auftritt.

Diese Aufgabe wird erfindungsgemäss dadurch gelöst, dass aus erntefrischen Pflanzenteilen ein Saft gepresst wird, der zur Zerstörung der Enzymaktivität einer Kurzzeiterhitzung unterworfen wird, dass dem Frischpflanzensaft zur Matrixbildung bis zu 20% Mono- und/oder Disaccheride zugesetzt wird, dass der Frischpflanzensaft anschliessend getrocknet und pulverisiert wird und dass das Frischplanzensaftpulver mit einer Standardbrauserezeptur gemischt und zu einem Brausepräparat verarbeitet wird. Denkbar ist das Pressen zu Tabletten oder die Verarbeitung zu einem Granulat. Es hat sich überraschend gezeigt, dass getrocknete Frischpflanzensäfte bei Einarbeitung in ein Brausepräparat beim Auflösen nicht mehr verschleimen. Die trinkfertige Lösung hat ein appetitliches Aussehen und ist frei von Extraktrückständen oder dergleichen. Da für die Gewinnung der Trockensubstanz vorgängig keine Lösungsmittel verwendet wurden, liegen alle Wirkstoffe praktisch in der Form unverändert vor, wie sie in den Planzenzellen zu finden sind.

Bei der Herstellung des Frischpflanzenpressaftes aus den erntefrischen Pflanzenteilen findet vorteilhaft eine Zerkleinerung unter Schutzgas, eine sofortige Denaturierung der Proteine, sowie eine Keimzahlreduktion durch Uperisation statt. Dabei wird die Pflanze innert 10 bis max. 20 Stunden nach der Ernte verarbeitet und die Grobzerkleinerung findet unter relativ kühlen Bedingungen bei 10 bis 20° C und bei hoher Luftfeuchte bei 70 bis 100% rel. H statt. Die Pürierung der Pflanzenmasse findet unter Schutzgas, beispielsweise Stickstoff statt und der vollständige Aufschluss der Pflanzenzellen erfolgt vorzugsweise durch Vakuumextrusion unter hohem Vakuum von weniger als 10 mbar. Dagegen erfolgt die Dekantierung bei Ueberdruck von 1 bis 3 atm. über Filter gepresst, wobei der Rohsaft auf ca. 10° C gekühlt wird. Innerhalb von weniger als 120 Minuten wird der Rohsaft uperisiert (UHT-Verfahren) und durch eine dreistufige Filtration geklärt. Die Trocknung des Frischpflanzensaftes erfolgt dann vorteilhaft innerhalb von 24 Stunden nach der Gewinnung, spätestens aber innerhalb von 48 Stunden nach der Pressung.

Der Zusatz von Rosmarinextrakt als Antioxidans hat sich besonders bewährt, namentlich Rosmarinextrakt aus überkritischer Kohlendioxidextraktion.

Durch das Verfahren wird verhindert, dass eine Wirkstoffveränderung durch Oxidation, Hydrolyse oder durch bakteriologische Einwirkungen stattfinden kann.

Besonders vorteilhaft wird der Frischpflanzensaft vor dem Trocknen zentrifugiert. Dadurch können Trübstoffe und andere Festpartikel ausgeschieden werden. Die Trocknung des Frischpflanzensaftes erfolgt vorteilhaft innerhalb von 24 Stunden. spätestens aber innerhalb von 48 Stunden nach der Pressung. Dadurch wird verhindert, dass Wirkstoffveränderungen durch Oxidation oder durch bakteriologische Einwirkungen stattfinden können. Die frischen Säfte können aber auch ohne weiteres durch Alkoholzusatz oder auf andere Weise konserviert werden, um dann erst bei Bedarf getrocknet zu werden.

Die Trocknung des Frischpflanzensaftes kann vorteilhaft in einem Vakuumbandtrockner erfolgen und zwar bei einer Temperatur von weniger als 50° C und bei einem reduzierten Druck von 6 bis 15 mbar. Vakuumbandtrockenverfahren sind dem Fachmann grundsätzlich bekannt und werden daher hier nicht näher beschrieben. Der Zusatz von bis zu 20% Mono- und/oder Disacchariden, insbesondere von Maltodextrin oder Lactose im Vakuumbandverfahren ergibt jedoch besonders kompakte, glasartig amorphe Strukturen, die sich durch ausgeprägte Stabilisierung der Wirkstoffstrukturen auszeichnen.

Zum Kaschieren des teilweise unangenehmen Geschmackes der Frischpflanzenkonzentrate können zusätzliche Geschmacks- und Geruchskorrigentien in das Frischpflanzensaftpulver oder in die fertigen Darreichungsformen eingearbeitet werden. Ebenso können zum Erzielen synergistischer Effekte zusätzlich Vitamin- und/oder Mineralien und/oder Spurenelemente und/oder Provitamine eingearbeitet werden. Auch die Einarbeitung zusätzlicher pharmakologischer Wirkstoffe auf synthetischer oder natürlicher Basis, rein oder in Form von Extrakten oder Konzentraten kann in bestimmten Fällen sinnvoll sein.

Die Erfindung betrifft auch ein vorzugsweise nach dem genannten Verfahren hergestelltes Brausepräparat, welches sich durch verschiedene Eigenschaften auszeichnet. Der Anteil an getrocknetem Frischpflanzensaft vom Gesamtgewicht des Brausepräparats kann dabei von 0,1% bis 50% schwanken. Damit können ggf. auch sehr hohe Wirkstoffkonzentrationen bei einer noch vertretbaren Auflösezeit des Brausepräparats pro Einzeldosis verabreicht werden.

Eine besonders vorteilhafte Aufnahme der Wirkstoffe im Körper ist gewährleistet, wenn das Brausepräparat in Wasser eine isotonische Lösung ergibt.

Die Herstellung des Brausepräparats kann auf unterschiedliche Weise erfolgen, wobei in der Regel eine Kombination von Bicarbonaten und Carbonaten und einem sauern Anteil eingesetzt wird. Basisrezepturen für Brausepräparate sind dem Fachmann jedoch bereits bekannt und in den entsprechenden Lehrbüchern beschrieben. Je nach Bedarf kann das Präparat auch noch ein Bindemittel enthalten.

Die Brausepräparate können unter Einarbeitung von getrockneten Frischpflanzensäften hergestellt werden, die auf einen bestimmten Wirkstoff oder auf eine Leitsubstanz standardisiert sind. Grundsätzlich lassen sich alle pressbaren Pflanzenteile (Herba, Radix, Flos, Fructus, Cortex) auf die erfindungsgemässe Art zu Frischpflanzensaftpulver verarbeiten, wobei es sich um Arzneipflanzen oder auch um solche Pflanzen handeln kann, die normalerweise lediglich zu Genuss- oder Nahrungsmitteln verarbeitet werden. Auch reine Aromastoffe können durch die Pressung gewonnen und in ein Brausepräparat eingearbeitet werden. Gemüsesäfte können sowohl als reine Nährmittel, als auch zu diätetischen Zwecken verarbeitet werden.

Im pharmazeutischen Bereich wurden besonders gute Resultate mit der Verarbeitung von Sonnenhut, Ginkgo oder Weissdorn erzielt. Artischocken können zu einem Arzneimittel oder zu einem Aperitifgetränk aufbereitet werden. Insbesondere beim Einsatz der Brausepräparate für die Zubereitung von Erfrischungsgetränken ist selbstverständlich auch der Einsatz von Lebensmittelfarbstoffen denkbar.

Ein paar Ausführungsbeispiele der Erfindung sind nachstehend wiedergegeben:
- Beispiel 1:: 200 mg Frischpflanzensaftpulver hergestellt aus Echinacea angustifolia und/oder Echinacea pallida und/-oder Echinacea purpurea unter Zusatz von 20 bis 40 mg Dextrin und Lactose und 0,1 mg Rosmarinextrakt im Vakuumbandtrockner bei weniger als 50° C 6 bis 15 mbar Druck getrocknet und anschliessend mit einer Standardbrauserezeptur gemischt und auf geeigneten Pressen zu Tabletten von 4,5 g Endgewicht gepresst.
- Beispiel 2:: Gleich wie Beispiel 1 aber mit einem Zusatz von 200 mg Vitamin C.
- Beispiel 3:: Standardbrauserezeptur mit 200 mg getrocknetem Frischpflanzensaftpulver aus Echinacea pallida herba und/oder purpurea und/oder angustifolia werden gemäss Beispiel 2 verarbeitet, wobei der Mischung zusätzlich 600 mg MgCO₃ zugefügt wird.
- Beispiel 4:: Standardbrauserezeptur mit 200 mg getrocknetem Frischpflanzensaftpulver aus Echinacea pallida herba und/oder purpurea und/oder angustifolia werden gemäss Beispiel 2 verarbeitet, wobei der Mischung 1000 mg CaCo zugefügt wird. Das Endgewicht der Tabletten beträgt 6,5 g.
- Beispiel 5:: Standardbrauserezeptur wird mit 500 mg getrocknetem Frischpflanzensaftpulver aus Artischocken (Blätter oder Früchte) verarbeitet. Das Endgewicht der Tabletten beträgt 4,5 g.
Beispiel 6: Standardbrauserezeptur wird mit 300 mg getrocknetem Frischpflanzensaftpulver aus Ginkgo-Blätter verarbeitet.
Beispiel 7: Standardbrauserezeptur wird mit 150 mg getrocknetem Frischpflanzensaftpulver aus Weissdorn (Blüten, Blätter oder Früchte) mit Vitamin E und 500 mg MgCO verarbeitet. Das Endgewicht der Tabletten beträgt 4,5 g.
Beispiel 8: Standardbrauserezeptur wird mit 150 mg getrocknetem Frischpflanzensaftpulver aus Weissdorn (Blüten, Blätter oder Früchte) gemäss Beispiel 7 verarbeitet, wobei der Mischung 30 mg Coenzym Q10 zugefügt wird.
Beispiel 9: Standardbrauserezeptur wird mit 200 mg getrocknetem Frischpflanzensaftpulver aus Johanniskraut verarbeitet.
Beispiel 10: Standardbrauserezeptur wird mit 200 mg getrocknetem Frischpflanzensaftpulver aus Echinacea pallida herba und/oder purpurea und/oder angustifolia und mit 500 mg Paracetamol und mit 250 mg Vitamin C verarbeitet.
Beispiel 11: Standardbrauserezeptur wird mit 200 mg getrocknetem Frischpflanzensaftpulver aus Echinacea pallida herba und/oder purpurea und/oder angustifolia verarbeitet, wobei der Mischung Coffein beigefügt wird.

## Patentansprüche

1. Verfahren zum Herstellen eines Brausepräparats als Träger eines Arzneimittels oder Genussmittels auf pflanzlicher Basis, dadurch gekennzeichnet,
- dass aus erntefrischen Pflanzenteilen ein Saft gepresst wird, der zur Zerstörung der Enzymaktivität einer Kurzzeiterhitzung unterworfen wird,
- dass dem Frischpflanzensaft zur Matrixbildung bis zu 20% Mono- und/oder Disaccharide zugesetzt wird,
- dass der Frischpflanzensaft anschliessend getrocknet und pulverisiert wird,
- und dass das Frischpflanzensaftpulver mit einer Standardbrauserezeptur gemischt und zu einem Brausepräparat verarbeitet wird.

2. Verfahren nach Anspruch 1, dadurch gekennzeichnet, dass die erntefrischen Pflanzenteile durch eine Vakuumextrusion aufgeschlossen werden.

3. Verfahren nach Anspruch 1 oder 2, dadurch gekennzeichnet, dass der Frischpflanzensaft vor dem Trocknen zentrifugiert wird.

4. Verfahren nach einem der Ansprüche 1 bis 3, dadurch gekennzeichnet, dass dem Frischpflanzensaft als Antioxidans Rosmarinextrakt zugesetzt wird.

5. Verfahren nach einem der Ansprüche 1 bis 4, dadurch gekennzeichnet, dass die Trocknung des Frischpflanzensaftes im Vakuumbandtrockenverfahren bei weniger als 50° C Temperatur und bei 6 bis 15 mbar Druck erfolgt.

6. Verfahren nach einem der Ansprüche 1 bis 5, dadurch gekennzeichnet, dass als hydrophobe Matrix langkettige Triglyzeride und Zusatz von Wachsen verwendet wird.

7. Brausepräparat als Träger eines Arzneimittels oder Genussmittels auf pflanzlicher Basis, insbesondere hergestellt nach dem Verfahren nach Anspruch 1, gekennzeichnet durch einen Anteil Frischpflanzensaftpulver aus erntefrischen Pflanzenteilen vom Gesamtgewicht des Brausepräparats von 0,1% bis 50%.

8. Brausepräparat nach Anspruch 7, gekennzeichnet durch zusätzliche Geschmacks- und Geruchskorrigentien.

9. Brausepräparat nach Anspruch 7 oder 8, gekennzeichnet durch zusätzliche Vitamine und/oder Mineralien und/oder Spurenelemente und/oder Provitamine.

10. Brausepräparat nach einem der Ansprüche 7 bis 9, gekennzeichnet durch zusätzliche pharmakologische Wirkstoffe.

11. Brausepräparat nach einem der Ansprüche 7 bis 10, dadurch gekennzeichnet, dass es eine der Pflanzengattungen Artischoke (Cynara scolymus), Sonnenhut (Echinacea Arten), Ginkgo (Ginkgo biloba), Weissdorn (Crataegus Arten), Johanniskraut (Hypericum), Zinnkraut (Herba equisetum) oder Löwenzahn (Taraxacum) enthält.

12. Brausepräparat nach einem der Ansprüche 7 bis 11, dadurch gekennzeichnet, dass es in Wasser eine isotonische Lösung ergibt.

## Claims

1. A method for manufacturing a plant-based effervescent preparation as a carrier of a medicine or stimulant, characterised in that
- from freshly harvested plant parts there is pressed a juice which is subjected to a short-lived heating for destroying the enzyme activity,
- that to the fresh plant juice for matrix formation up to 20% mono- and/or disaccharides are added,
- that the fresh plant juice is subsequently dried and pulverised,
- and that the fresh plant juice powder is mixed with a standard effervescent recipe and processed into an effervescent preparation.

2. A method according to claim 1, characterised in that the freshly harvested plant parts are opened up by vacuum extrusion.

3. A method according to claim 1 or 2, characterised in that the fresh plant juice is centrifuged before the drying.

4. A method according to one of the claims 1 to 3, characterised in that to the fresh plant juice as an antioxidant rosemary extract is added.

5. A method according to one of the claims 1 to 4, characterised in that the drying of the fresh plant juice with the vacuum drying method is effected at less than 50° C temperature and at 6 to 15 mbar pressure.

6. A method according to one of the claims 1 to 15, characterised in that as a hydrophobic matrix, long-chain triglycerides and the addition of waxes is used.

7. A plant-based efffervescent preparation as a carrier of a medicine or stimulant, in particular manufactured according to a method according to claim 1, characterised by a constituent part of fresh plant juice powder of freshly harvested plant parts of 0.1 % to 50% of the total weight of the effervescent preparation.

8. An effervescent preparation according to claim 7, characterised by additional taste and odour improvers.

9. An effervescent preparation according to claim 7 or 8, characterised by additional vitamins and/or minerals and/or trace elements and/or provitamins.

10. An effervescent preparation according to one of the claims 7 to 9, characterised by additional pharmacological active ingredients.

11. An effervescent preparation according to one of the claims 7 to 10, characterised in that it contains one of the plant species artichoke (Cynara scolymus), coneflower (Echinacea Arten), Ginko (Ginko bilboa), whitethorn (Crataegus Arten), St Johns wort (Hypericum), horsetail (Herba equisetum) or dandelion (Taraxacum).

12. An effervescent preparation according to one of the claims 7 to 11, characterised in that it in water yields an isotonic solution.

## Revendications

1. Procédé pour fabriquer une préparation effervescente comme support d'un médicament ou d'un stimulant à base de plantes, caractérisé
- en ce qu'on extrait de parties de plantes fraîchement cueillies un jus qui est soumis à un bref réchauffement pour détruire l'activité enzymatique,
- en ce qu'on ajoute jusqu'à 20 % de monosaccharides et/ou de disaccharides au jus de plante frais, pour former la matrice,
- en ce qu'on sèche et on pulvérise ensuite le jus de plante frais,
- et en ce qu'on mélange le jus de plante frais en poudre avec une composition effervescente standard et on le transforme en préparation effervescente.

2. Procédé selon la revendication 1, caractérisé en ce que les parties de plantes fraîchement cueillies sont décomposées par extrusion sous vide.

3. Procédé selon la revendication 1 ou 2, caractérisé en ce que le jus de plante frais est centrifugé avant le séchage.

4. Procédé selon l'une des revendications 1 à 3, caractérisé en ce qu'on ajoute au jus de plante frais, comme agent antioxydant, de l'extrait de romarin.

5. Procédé selon l'une des revendications 1 à 4, caractérisé en ce que le séchage du jus de plante frais se fait selon le procédé du séchage à bande sous vide, à une température inférieure à 50°C et avec une pression de 6 à 15 mbar.

6. Procédé selon l'une des revendications 1 à 5, caractérisé en ce qu'on utilise comme matrice hydrophobe des triglycérides à longue chaîne avec une adjonction de cires.

7. Préparation effervescente comme support d'un médicament ou d'un stimulant à base de plantes, fabriquée en particulier à l'aide du procédé selon la revendication 1, caractérisée en ce qu'elle contient une proportion de jus de plante frais en poudre provenant de parties de plantes fraîchement cueillies qui est de 0,1 % à 50 % par rapport au poids total de la préparation effervescente.

8. Préparation effervescente selon la revendication 7, caractérisée en ce qu'elle comprend en outre des correctifs de goût et d'odeur.

9. Préparation effervescente selon la revendication 7 ou 8, caractérisée en ce qu'elle comprend en outre des vitamines et/ou des minéraux et/ou des éléments à l'état de traces et/ou des provitamines.

10. Préparation effervescente selon l'une des revendications 7 à 9, caractérisée en ce qu'elle comprend en outre des substances actives pharmacologiques.

11. Préparation effervescente selon l'une des revendications 7 à 10, caractérisée en ce qu'elle contient l'une des espèces de plantes suivantes : artichaut (Cynara scolymus), rudbeckia (genre Echinacea), Ginkgo (Ginkgo biloba), aubépine (genre Crataegus), millepertuis (Hypericum), prêle des champs (Herba equisetum) ou pissenlit (Taraxacum).

12. Préparation effervescente selon l'une des revendications 7 à 11, caractérisée en ce qu'elle donne, dans l'eau, une solution isotonique.
